Europäisches Patentamt

(19) European Patent Office

Office européen des brevets

(11) Veröffentlichungsnummer: **0 014 971**
**B1**

(12) **EUROPÄISCHE PATENTSCHRIFT**

(45) Veröffentlichungstag der Patentschrift:
16.06.82

(51) Int. Cl.³: **C 12 P 33/06, C 07 J 1/00 //**
**C12R1/66, C12R1/80**

(21) Anmeldenummer: 80100768.3

(22) Anmeldetag: 14.02.80

(54) Verfahren zur Herstellung von 1-alpha-Hydroxydehydroepiandrosteron.

(30) Priorität: 16.02.79 CH 1540/79

(43) Veröffentlichungstag der Anmeldung:
03.09.80 Patentblatt 80/18

(45) Bekanntmachung des Hinweises auf die Patenterteilung:
16.06.82 Patentblatt 82/24

(84) Benannte Vertragsstaaten:
CH DE FR GB IT

(56) Entgegenhaltungen:
FR-A-2 287 909
GB-A-809 494
US-A-2 833 793
US-A-2 833 794
JOURNAL OF THE AMERICAN CHEMICAL SOCIETY,
Band 79, 20. Juli 1957, Columbus, Ohio, US,
R.M. DODSON et al.: «Microbiological hydroxylation
of C19-steroids at positions C-1 and C-2», Seite 3921

(73) Patentinhaber: F. HOFFMANN-LA ROCHE & CO.
Aktiengesellschaft, CH-4002 Basel (CH)

(72) Erfinder: Fujiwara, Akiko, 1059-7 Fueta, Kamakura-shi
Kanagawa-ken (JP)
Erfinder: Miyamoto, Chikara, 505 Marumo
Building 250 Kuden-cho Totsuka-ku, Yokohama-shi
Kanagawa-ken (JP)
Erfinder: Okuda, Toru, 74 Yakuoji-machi Ichigaya,
Shinjuku-ku Tokyo (JP)

(74) Vertreter: Wuesthoff, Franz, Dr.-Ing. et al, Patentanwälte
Wuesthoff -v. Pechmann-Behrens-Goetz
Schweigerstrasse 2, D-8000 München 90 (DE)

## Verfahren zur Herstellung von 1α-Hydroxydehydroepiandrosteron

Die vorliegende Erfindung betrifft ein mikrobiologisches Verfahren zur Herstellung von 1α-Hydroxydehydroepiandrosteron, dadurch gekennzeichnet, dass man Dehydroepiandrosteron oder ein Derivat davon mit einem Mikroorganismus der Spezies Penicillium oxalicum oder Aspergillus terreus, der zur 1α-Hydroxylierung von Dehydroepiandrosteron oder einem Derivat davon befähigt ist, oder mit aus einer Kultur der genannten Mikroorganismen isoliertem Mycel oder mit einem in an sich bekannter Weise aus der Kulturlösung oder dem Mycel hergestellten Enzymextrakt zu einem Zeitpunkt zusammenbringt, an dem die Kohlenstoffquelle des Kulturmediums fast vollständig aufgebraucht ist.

Aus der japanischen Patentanmeldung Kokai No. 63990/1976 ist bekannt, 1α,17β-Dihydroxysteroide durch 1α-Hydroxylierung von 17β-Hydroxy-17α-substituierten oder unsubstituierten 4-Oestren-3-onen mittels Mikroorganismen der Genera Rhizoctonia, Calonectria, Glomerella, Aspergillus, Corticium, Septomyxa, Mucor, Isaria, Irpex oder Fusarium herzustellen. Alle diese Mikroorganismen mit Ausnahme der Spezies Aspergillus terreus sind nicht in der Lage, Dehydroepiandrosteron in 1α-Stellung zu hydroxylieren. Dieser Umstand bedeutet, dass eine 1α-Hydroxylierung von Steroiden nur mit ganz bestimmten Kombinationen von Mikroorganismen und Substraten durchgeführt werden kann. Es ist in der Tat sehr schwierig, eine solche Kombination herauszufinden. Nach dem Verfahren der oben erwähnten Patentanmeldung können Aspergillus clavatus ATCC 9598, A. fumigatus mut. helvola CBS 110.46 und A. conics IFO 4047 Steroide mit einer Oxogruppe in 3-Stellung, einer Doppelbindung in der 4,5-Stellung und einer Hydroxygruppe in 17β-Stellung in 1α-Stellung hydroxylieren. Diese Mikroorganismen können jedoch Dehydroepiandrosteron, das in 3-Stellung eine Hydroxygruppe, eine 5,6-Doppelbindung und eine 17-Oxogruppe aufweist, oder Derivate davon, nicht in 1α-Stellung hydroxylieren.

Aus der US-Patentschrift 2 883 794 ist bekannt, Dehydroisoandrosteron (Dehydroepiandrosteron) mittels Penicillium sp. ATCC 12556 in 1α-Stellung zu hydroxylieren. Dieses bekannte Verfahren besitzt den Nachteil, dass dabei viel niedrigere Substratkonzentrationen, nämlich etwa 0,35 g/l, eingesetzt werden. Es ist daher schwierig, 1α-Hydroxy-dehydroepiandrosteron nach der in der US-Patentschrift 2 883 794 beschriebenen Arbeitsweise in hoher Ausbeute herzustellen, dies um so mehr, als sich bei der angewandten niedrigen Substratkonzentration das Hydroxylierungsprodukt unmittelbar nach Erreichen der höchsten Ausbeute zu zersetzen scheint.

Es wurde nun gefunden, dass man 1α-Hydroxydehydroepiandrosteron in hoher Ausbeute durch Hydroxylierung von Dehydroepiandrosteron oder einem Derivat davon mittels geeigneter Mikroorganismen der Spezies Penicillium oxalicum oder Aspergillus terreus herstellen kann, wenn man das Substrat der Kulturlösung der genannten Mikroorganismen zu einem Zeitpunkt zusetzt, an dem die Kohlenstoffquelle der Nährlösung fast vollständig verbraucht ist.

Als Derivate des Dehydroepiandrosterons kommen für den erfindungsgemässen Zweck vor allem 3-Acylderivate, insbesondere das 3-Acetylderivat, in Betracht.

Das erfindungsgemässe Verfahren kann mit allen Stämmen der Spezies Penicillium oxalicum oder Aspergillus terreus durchgeführt werden, die zu 1α-Hydroxylierung von Dehydroepiandrosteron oder einem Derivat davon befähigt sind. Bevorzugte Stämme sind Penicillium oxalicum IFO-5748 (FERM-P. No. 4727; ATCC 20590), P. oxalicum IFO-6579 (FERM-P No. 4728; ATCC 20591), P. Oxalicum IFO-7000 (FERM-P No. 4729; ATCC 20592), P. oxalicum IFO-7085 (FERM-P No. 4730; ATCC 20593) und Aspergillus terreus IFO-6123 (FERM-P No. 4726; ATCC 20589).

Die Bezeichnung IFO bezieht sich auf Kulturen des Institute for Fermentation, Osaka, Japan; die Bezeichnung FERM-P bezieht sich auf Kulturen des Fermentation Research Institute, Chiba city, Japan und ATCC bezeichnet die American Type Culture Collection, Rockville, Maryland, USA.

Die folgenden Stämme des Genus Penicillium und Aspergillus können Dehydroepiandrosteron oder ein Derivat davon nicht hydroxylieren: P. verruculosum, P. notatum, P. chrysogenum, P. decumbens, P. fellutanum, P. frequentans, P. purpurrescens, P. terlikowskii, P. lilacino-echinulatum, P. multicolor, P. simplicissimum, P. herquei, P. implicatum, P. janthinellum, P. charlesii, P. citreo-viride, P. corylophiloides, P. cyaneum, P. adametzi, P. aeneum, P. thomii, und P. sclerotiorum: A. flavus, A. ustus, A. clavatus, A. parasiticus, A. conicus, A. carneus und A. fumigatus.

Der Mikroorganismus kann in Form der Kulturlösung, des Mycels oder in aufbereiteter Form verwendet werden. Die Kulturlösung kann durch Beimpfen eines geeigneten Mediums mit dem Mikroorganismus hergestellt werden. Das Kulturmedium kann Kohlenstoffquellen, Stickstoffquellen, anorganische Salze und andere für das Wachstum des Mikroorganismus geeignete Nährstoffe enthalten. Kohlenstoffquellen sind z.B. Glucose, Sucrose, Dextrin, Mannose, Stärke, Lactose und Glycerin; Stickstoffquellen sind beispielsweise stickstoffhaltige organische Substanzen, wie Peptone, Fleischextrakt, Hefeextrakt, Maisquellwasser und Casein, oder stickstoffhaltige anorganische Verbindungen, wie Nitrate und anorganische Ammoniumsalze. Beispiele anorganischer Salze sind Phosphate oder Natrium-, Kalium-, Mangan-, Magnesium-, Eisen- und Kupfer-Salze.

Die Kultivierung des Mikroorganismus kann als Submerskultur, als Schüttelkultur oder als stationäre Kultur erfolgen. Vorzugsweise wird der Mikroorganismus unter aeroben Bedingungen kultiviert.

Das erfindungsgemässe Verfahren wird zweckmässig dadurch ausgeführt, dass man Dehydroepiandrosteron oder ein Derivat davon der Nährlösung zusetzt. Die Konzentration des Substrats beträgt zweckmässig 1–15 g/l, vorzugsweise 1–10 g/l. Die 1α-Hydroxylierung kann dadurch ausgeführt werden, dass man die Kultivierung des Mikroorganismus unter den oben erwähnten Bedingungen fortsetzt. Die Kultivierungszeit kann vom verwendeten Mikroorganismus, von der Zusammensetzung des Kulturmediums, von dem verwendeten Substrat und seiner Konzentration abhängen. Im allgemeinen genügt eine Kultivierungszeit von 1–10 Tagen. Die Kultivierungstemperatur liegt im allgemeinen zwischen 20 und 30 °C. Zweckmässig wird die Kultivierung bei einem pH zwischen 4 und 9 ausgeführt.

Der Zeitpunkt des Zusatzes des Substrates zur Kulturlösung ist wichtig, um eine hohe Ausbeute an 1α-Hydroxydehydroepiandrosteron zu erhalten. Das erfindungsgemässe Verfahren, bei dem der Zusatz zur Nährlösung erfolgt, wenn die Kohlenstoffquelle beinahe vollständig verbraucht ist, führt zu einer hohen Ausbeute.

Der Zeitpunkt, an dem die Kohlenstoffquelle in der Nährlösung beinahe vollständig aufgebraucht ist, kann z.B. durch Bestimmung des Glucosegehalts der Nährlösung festgestellt werden. Der Zusatz des Steroids kann z.B. erfolgen, wenn der Glucosegehalt der Nährlösung auf 0,05 bis 0,02% gesunken ist. Da das Wachstum des Mikroorganismus stagniert, wenn die Kohlenstoffquelle verbraucht ist, kann der Zeitpunkt des Steroidzusatzes auch durch Beobachtung der Änderung der Zellmasse der Mikroorganismen pro Volumeneinheit (z.B. g Trockenzellen/Liter Fermentationslösung) ermittelt werden. Das Steroid wird zweckmässig zugesetzt, wenn das Trockenzellgewicht pro Volumeneinheit nicht mehr zunimmt. Dieser Zeitpunkt hängt von den Fermentationsbedingungen ab und kann z.B. nach 2 Tagen erreicht sein.

Alternativ kann die erfindungsgemässe 1α-Hydroxylierung auch mit dem aus der Kulturlösung des Mikroorganismus isolierten Mycel oder mit einem in an sich bekannter Weise aus der Kulturlösung oder dem Mycel hergestellten Enzymextrakt ausgeführt werden. In diesem Falle wird die 1α-Hydroxylierung zweckmässig in Lösung, z.B. in einer Pufferlösung, in einer physiologischen Salzlösung, in frischer Nährlösung oder in Wasser ausgeführt.

Das Substrat, Dehydroepiandrosteron oder ein Derivat davon, kann in Form eines feinen Pulvers oder in Form einer Lösung, in einem hydrophilen Lösungsmittel, wie Aceton, Dimethylsulfoxid, Methanol, Äthanol, Äthylenglykol, Propylenglykol oder Dioxan, zugesetzt werden. Man kann auch ein oberflächenaktives oder Dispersionsmittel einer wässrigen Suspension des Substrats zusetzen oder das Substrat kann durch Behandlung mit Ultraschall emulgiert werden.

Auch wenn ein Derivat des Dehydroepiandrosterons, wie das Dehydroepiandrosteron-3-acetat, im erfindungsgemässen Verfahren eingesetzt wird, kann 1α-Hydroxydehydroepiandrosteron erhalten werden.

Das so erhaltene 1α-Hydroxydehydroepiandrosteron kann aus dem Fermentationsgemisch oder dem Reaktionsgemisch in an sich bekannter Weise isoliert werden, beispielsweise durch Solventextraktion mit einem mit Wasser nicht mischbaren organischen Lösungsmittel, wie Chloroform, Methylenchlorid oder Äthylacetat, oder durch Chromatographie auf einem Träger, wie Aluminiumoxid, Silicagel oder Cellulose. Das so isolierte Produkt kann beispielsweise durch Umkristallisation aus Methanol, Äthanol, Äthylacetat, Benzol oder Aceton gereinigt werden.

Das erfindungsgemäss hergestellte 1α-Hydroxydehydroepiandrosteron ist eine bekannte Verbindung, die als Zwischenprodukt zur Herstellung von Pharmazeutika Verwendung finden kann.

Die nachfolgenden Beispiele erläutern die Erfindung. Die Temperaturen sind in Celsiusgraden angegeben.

Beispiel 1

Ein 500 ml-Kolben mit 100 ml eines Mediums, enthaltend 1% Glucose und 1% Maisquellwasser wurde 20 Minuten bei 120° autoklaviert. Das pH des Mediums wurde zuvor auf 6,5 eingestellt. Nach Abkühlen wurde der Kolbeninhalt mit Sporen einer Schrägagarkultur von Penicillium oxalicum IFO-7000 (FERM-P No. 4729) beimpft.

Der Kolben wurde dann bei 26,5° 2 Tage auf einer Rotationsschüttelmaschine inkubiert. Dehydroepiandrosteron wurde in einer 0,1%igen Tween 80-Lösung unter Ultraschall suspendiert und dem Kolbeninhalt bis zu einer Endkonzentration von 5 g/l zugesetzt. Die Inkubation wurde 3 Tage unter den obigen Bedingungen fortgesetzt.

10 ml-Proben wurden entnommen und unter Schütteln mit drei Volumenteilen Äthylacetat extrahiert. Ein Teil des Extraktes wurde dünnschichtchromatographisch analysiert mit Silicagelplatten und einem Lösungsmittelsystem von Chloroform-Aceton (2:1) und Cyclohexan-Toluol-Äthanol (8:2:2,5), sowie durch Gasflüssigchromatographie. Die Ausbeute an 1α-Hydroxydehydroepiandrosteron betrug 45,0%.

Beispiel 2

In Analogie zu dem in Beispiel 1 beschriebenen Verfahren, bei dem das Substrat als Pulver in einer Endkonzentration von 5 g/l zugesetzt wurde, erhielt man 1α- Hydroxydehydroepiandrosteron in 30,2% Ausbeute.

Beispiel 3

In Analogie zu dem in Beispiel 1 beschriebenen Verfahren erhielt man mit Substratkonzentrationen von 3 g/l und 7 g/l 1α-Hydroxydehydroepiandrosteron in Ausbeuten von 36,3% und 26,5%.

Beispiel 4

In Analogie zu dem in Beispiel 1 beschriebenen Verfahren erhielt man mit einer Substratkonzentration von 3 g/l Dehydroepiandrosteron-3-acetat

1α-Hydroxydehydroepiandrosteron in 32% Ausbeute.

## Beispiel 5

In Analogie zu dem Verfahren von Beispiel 1 erhielt man unter Verwendung von Penicillium oxalicum IFO-5748 (FERM-P No. 4727), IFO-6579 (FERM-P No. 4728) und IFO-7085 (FERM-P No. 4730) 1α-Hydroxydehydroepiandrosteron in Ausbeuten von 24,0%, 23,2% und 32,0%.

## Beispiel 6

Ein 500 ml-Kolben mit 100 ml eines Mediums enthaltend 1% Glucose und 1% Maisquellwasser wurde mit Sporen von Aspergillus terreus IFO-6123 (FERM-P No. 4726) beimpft und 2 Tage bei 26,5° geschüttelt. 100 mg Dehydroepiandrosteron wurden in 2 ml 0,1%iger Tween 80-Lösung unter Ultraschall suspendiert und dem Inhalt des Kolbens zugesetzt. Die Kultivierung wurde 2 Tage fortgesetzt, worauf man aus dem Medium 1α-Hydroxydehydroepiandrosteron isolierte und identifizierte.

## Beispiel 7

Ein 500 ml-Kolben mit 100 ml vorsterilisiertem Medium (pH 6,5) enthaltend 1% Glucose und 1% Maisquellwasser wurde mit einer Schrägkultur von Penicillum oxalicum IFO-7000 (FERM-P No. 4729) beimpft und 2 Tage bei 26,5° geschüttelt. Ein 5 Liter-Fermentationsgefäss mit 3 Liter vorsterilisiertem Medium wurde mit der so erhaltenen Kultur beimpft. Die Fermentation wurde 37 Stunden und bei 26,5° mit Rühren (300 rpm) und Belüftung (1 v/v) fortgesetzt.

Eine Suspension von 15 g Dehydroepiandrosteron in 300 ml 0,1%igem Tween 80 wurde 10 Minuten beschallt und dem Fermentationsgefäss zugesetzt. Die Fermentation wurde 79 Stunden unter den obigen Bedingungen fortgeführt. Die Produktion an 1α-Hydroxydehydroepiandrosteron im Nährmedium betrug 5,36 g.

Die Kultur wurde geerntet, mit 3 l Äthylacetat extrahiert und das Mycel wurde abfiltriert. Der wässrige Acetonextrakt des Mycels wurde zur Entfernung des Acetons eingeengt und mit Äthylacetat extrahiert. Die wässrige Phase der ersten Extraktion wurde ebenfalls mit Äthylacetat extrahiert. Die vereinigten Extrakte wurden unter vermindertem Druck auf ein kleines Volumen konzentriert. 3,82 g Feststoff wurden abfiltriert. Kristalisation des Feststoffes aus einem Gemisch von Methanol und Äthylacetat lieferten 2,56 g gereinigtes 1α-Hydroxydehydroepiandrosteron vom Schmelzpunkt ca. 279°.

Die Mutterlauge aus der Filtration und der Kristallisation wurde zur Trockene eingedampft und auf einer Silicagel-Säule chromatographiert. Die Säule wurde mit einem Gemisch von Aceton und Chloroform eluiert. Während der Elution wurden die Anteile an Aceton nach und nach erhöht. Man erhielt so 1,5 g 1α-Hydroxydehydroepiandrosteron vom Schmelzpunkt 278,5°.

## Beispiel 8

Zehn 500 ml-Kolben mit jeweils 100 ml Nährmedium (1% Glucose, 1% Maisquellwasser, pH 6,5) wurden mit Sporen einer Schrägkultur von Penicillium oxalicum IFO-7000 (FERM-P No. 4729) beimpft und unter Schütteln bei 26,5° 2 Tage lang kultiviert. Das Mycel wurde durch Filtration gesammelt, und mit sterilem Wasser gewaschen. Das gewaschene Mycel wurde in einen 5 Liter-Kolben mit 1 Liter 0,85%iger Salzlösung, 10 g CaCO₃ und 1 g Dehydroepiandrosteron gegeben. Der Kolben wurde unter Schütteln 20 Stunden bei 26,5° inkubiert. Danach waren 35% des Dehydroepiandrosterons in die entsprechende 1α-Hydroxy-Verbindung überführt worden. Das Reaktionsgemisch wurde mit 1 Liter Äthylacetat extrahiert und das Mycel abfiltriert. Die organische Phase wurde über Natriumsulfat getrocknet und zu einem Feststoff konzentriert, der dann auf eine Silicagel-Säule gegeben wurde. Die Säule wurde mit einem Gemisch von Aceton und Chloroform eluiert, wobei während der Elution die Aceton-Anteile nach und nach erhöht wurden. Man erhielt so 230 mg reines 1α-Hydroxydehydroepiandrosteron vom Schmelzpunkt 278,5°.

## Patentansprüche

1. Verfahren zur mikrobiologischen Herstellung von 1α-Hydroxydehydroepiandrosteron, dadurch gekennzeichnet, dass man Dehydroepiandrosteron oder ein Derivat davon mit einem Mikroorganismus der Spezies Penicillium oxalicum oder Aspergillus terreus, der zur 1α-Hydroxylierung von Dehydroepiandrosteron oder einem Derivat davon befähigt ist, oder mit aus einer Kultur der genannten Mikroorganismen isoliertem Mycel oder mit einem in an sich bekannter Weise aus der Kulturlösung oder dem Mycel hergestellten Enzymextrakt zu einem Zeitpunkt zusammenbringt, an dem die Kohlenstoffquelle des Kulturmediums fast vollständig aufgebraucht ist.

2. Verfahren nach Anspruch 1, dadurch gekennzeichnet, dass der Mikroorganismus Penicillium oxalicum IFO-5748 (FERM-P No. 4727), Penicillium oxalicum IFO-6579 (FERM-P No. 4728), Penicillium oxalicum IFO-7000 (FERM-P No. 4729), Penicillium oxalicum IFO-7085 (FERM-P No. 4730) oder Aspergillus terreus IFO-6123 (FERM-P No. 4726) ist.

3. Verfahren nach den Ansprüchen 1 oder 2, dadurch gekennzeichnet, dass man als Substrat Dehydroepiandrosteron verwendet.

4. Verfahren nach Anspruch 1 oder 2, dadurch gekennzeichnet, dass man als Substrat Dehydroepiandrosteron-3-acetat verwendet.

5. Verfahren nach den Ansprüchen 1–4, dadurch gekennzeichnet, dass die Substratkonzentration 1–15 g/l beträgt.

6. Verfahren nach den Ansprüchen 1–5, dadurch gekennzeichnet, dass das Substrat mit dem Mycel in Lösung behandelt wird.

## Revendications

1. Procédé pour la préparation microbiologique de la 1α-hydroxydéshydroépiandrostérone, caractérisé en ce que l'on met en contact la déshydroépiandrostérone ou un dérivé de ce composé avec un microorganisme de l'espèce Penicillium oxalicum ou Aspergillus terreus, capable de provoquer la 1α-hydroxylation de la déshydroépiandrostérone ou d'un dérivé de ce composé, ou avec un mycélium isolé d'une culture des microorganismes mentionnés, ou avec un extrait enzymatique préparé de manière connue en soi à partir de la solution de culture ou du mycélium, à un moment auquel la source de carbone du milieu de culture est presque entièrement consommée.

2. Procédé selon la revendication 1, caractérisé en ce que le microorganisme est Penicillium oxalicum IFO-5748 (FERM-P No. 4727), Penicillium oxalicum IFO-6579 (FERM-P No. 4728), Penicillium oxalicum IFO-7000 (FERM-P No. 4729), Penicillium oxalicum IFO-7085 (FERM-P No. 4730) ou Aspergillus terreus IFO-6123 (FERM-P No. 4726).

3. Procédé selon l'une des revendications 1 et 2, caractérisé en ce que l'on utilise en tant que substrat la déshydroépiandrostérone.

4. Procédé selon l'une des revendications 1 et 2, caractérisé en ce que l'on utilise en tant que substrat le 3-acétate de la déshydroépiandrostérone.

5. Procédé selon l'une des revendications 1 à 4, caractérisé en ce que la concentration du substrat est de 1 à 15 g/l.

6. Procédé selon l'une des revendications 1 à 5, caractérisé en ce que l'on traite le substrat par le mycélium en solution.

**Claims**

1. Process for the microbiological manufacture of 1α-hydroxydehydroepiandrosterone, characterized by bringing together dehydroepiandrosterone or a derivative thereof with a microorganism of the species Penicillium oxalicum or Aspergillus terreus, which is capable of 1α-hydroxylating dehydroepiandrosterone or a derivative thereof, or with mycelium isolated from a culture of the said microorganisms or with an enzyme extract prepared in a manner known per se from the culture solution or the mycelium at a point in time at which the carbon source of the culture medium is almost completely consumed.

2. Process according to claim 1, characterized in that the microorganism is Penicillium oxalicum IFO-5748 (FERM-P No. 4727), Penicillium oxalicum IFO-6579 (FERM-P No. 4728), Penicillium oxalicum IFO-7000 (FERM-P No. 4729), Penicillium oxalicum IFO-7085 (FERM-P No. 4730) or Aspergillus terreus IFO-6123 (FERM-P No. 4726).

3. Process according to claims 1 or 2, characterized in that dehydroepiandrosterone is used as the substrate.

4. Process according to claim 1 or 2, characterized in that dehydroepiandrosterone-3-acetate is used as the substrate.

5. Process according to claims 1–4, characterized in that the substrate concentration amounts to 1–15 g/l.

6. Process according to claims 1–5, characterized in that the substrate is treated with the mycelium in solution.